# EUROPEAN PATENT APPLICATION

(11) **EP 2 090 891 A1**
(43) Date of publication of application: **19.08.2009**
(21) Application number: 08151376.4
(22) Date of filing: 13.02.2008
(51) Int. Cl.: G01N 33/68

(54) **Means and methods for determining the atherosclerotic load using the biomarker PLGF**

(71) Applicant: F.HOFFMANN-LA ROCHE AG, 4070 Basel (CH); Roche Diagnostics GmbH, 68305 Mannheim (DE)
(72) Inventor: Hess, Georg, Prof. Dr., 55130 Mainz (DE); Horsch, Andrea, Dr., 68259 Mannheim (DE); Zdunek, Dietmar, Dr., 82327 Tutzing (DE)
(74) Representative: Dick, Alexander

(57) **Abstract**

The present invention is concerned with the provision of diagnostic methods relating to atherosclerosis. Specifically, it relates to a method for diagnosing the arteriosclerotic load of a subject comprising determining the amount of PLGF in a sample of a subject and calculating the ratio of the determined amount and the upper limit of normal for PLGF, wherein a ratio of 1 indicates a normal arteriosclerotic load, a ratio less than 1 indicates a reduced arteriosclerotic load and a ratio larger than 1 indicates an increased arteriosclerotic load. The present invention also contemplates a method for identifying a subject in need of prevention or therapy of arteriosclerosis. Further, devices and kits are encompassed for carrying out said methods.

## Description

The present invention is concerned with the provision of diagnostic methods relating to atherosclerosis. Specifically, it relates to a method for diagnosing the arteriosclerotic load of a subject comprising determining the amount of PLGF in a sample of a subject and calculating the ratio of the determined amount and the upper limit of normal for PLGF, wherein a ratio of 1 indicates a normal arteriosclerotic load, a ratio less than 1 indicates a reduced arteriosclerotic load and a ratio larger than 1 indicates an increased arteriosclerotic load. The present invention also contemplates a method for identifying a subject in need of prevention or therapy of arteriosclerosis. Further, devices and kits are encompassed for carrying out said methods.

Atherosclerosis is a cardiovascular disease affecting the structure of the blood vessels. It is dependent on various risk factors including smoking, hyperlipidemia, arterial hypertension, or diabetes.

Atherosclerosis is a pathological process which in its advanced stages has severe complications caused by occlusions or stenosis of blood vessels. Prominent complications caused by the said stenosis or occlusion of blood vessels are coronary artery diseases especially angina pectoris, claudicato intermittens, myocardial infarction or stroke. These complications, however, become only clinically apparent if more than 90% of the vessel is occluded. Even in those cases they become often apparent only during exercise.

The risk of developing the aforementioned severe complications essentially depends on the atherosclerotic load within a subject, i.e. the overall amount of atherosclerotic plaques found in the subject. It is to be understood that most of the atherosclerotic plaques found in a subject will not result in any of the aforementioned complications. However, there is an increasing risk for developing harmful plaques as the overall amount of atherosclerotic plaques increases.

The atherosclerotic load is currently determined by cumbersome, expensive and/or invasive techniques including angiography. These techniques are inconvenient for the patient to be investigated and are time- and cost- intensive from an overall health management perspective. Moreover, invasive angiography may even result in severe side-effects for the patient (C.J. Davidson, R.O. Bonow Cornorary catherization p 345; D. Pennell Cardiovascular Magnetic Resonance p 335; S. Achenbach, W.G. Daniel Computed tomography of the heart p. 255 all in Braunwald's Heart Disese 7th Ed. 2005 Elevier Publishers)

Means and methods allowing for a reliable and efficient determination of the atherosclerotic load and, thus, or assessing the risk for developing severe complications of atherosclerosis referred to above are not yet available but would be highly desirable.

The technical problem underlying the present invention could be seen as the provision of means and methods for complying with the aforementioned needs. The technical problem is solved by the embodiments characterized in the claims and herein below.

Thus, the present invention relates to a method for diagnosing the atherosclerotic load of a subject comprising:
a) determining the amount of PLGF in a sample of a subject; and
b) calculating the ratio of the determined amount and the upper limit of normal for PLGF, wherein
   (i) a ratio of 1 indicates a normal atherosclerotic load;
   (ii) a ratio less than 1 indicates a reduced atherosclerotic load; and
   (iii) a ratio larger than 1 indicates an increased atherosclerotic load.

The method of the present invention, preferably, is an in vitro method. Moreover, it may comprise steps in addition to those explicitly mentioned above. For example, further steps may relate to sample pre-treatments or evaluation of the results obtained by the method. The method of the present invention may be also used for monitoring, confirmation, and subclassification of the subject. The method may be carried out manually or assisted by automation. Preferably, step (a) and/or (b) may in total or in part be assisted by automation, e.g., by a suitable robotic and sensory equipment for the determination in step (a) or a computer-implemented calculation in step (b).

The term "diagnosing" as used herein means assessing the atherosclerotic load in a subject. As will be understood by those skilled in the art, such an assessment is usually not intended to be correct for all (i.e. 100%) of the subjects to be investigated. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the subjects of a population can be properly assessed by the method of the present invention.

The term "atherosclerotic load" relates to the overall amount of atherosclerotic plaques found in a subject, i.e. the amount of atherosclerotic plaques found in the entire vessel system of the said subject. As a consequence of the atherosclerotic load, the risk for severe complications associated with atherosclerosis can be, preferably, diagnosed. More preferably, an increased arteriosclerotic load in this context further indicates an increased risk of developing angina pectoris, claudicato intermittens or stroke.

The term "subject" as used herein relates to animals, preferably mammals, and, more preferably, humans.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The term "PlGF (Placental Growth Factor)" as used herein refers to a placenta derived growth factor which is a 149-amino-acid-long polypeptide and is highly homologous (53% identity) to the platelet-derived growth factor-like region of human vascular endothelial growth factor (VEGF). Like VEGF, PlGF has angiogenic activity in vitro and in vivo. For example, biochemical and functional characterization of PlGF derived from transfected COS-1 cells revealed that it is a glycosylated dimeric secreted protein able to stimulate endothelial cell growth in vitro (Maqlione1993, Oncogene 8(4):925-31). Preferably, PlGF refers to human PlGF, more preferably, to human PlGF having an amino acid sequence as shown in Genebank accession number P49763, GI: 17380553 (Genebank is available from the NCBI, USA under www.ncbi.nlm.nih.gov/entrez). Such variants have at least the same essential biological and immunological properties as the specific PLGF polypeptides. In particular, they share the same essential biological and immunological properties if they are detectable by the same specific assays referred to in this specification, e.g., by ELISA assays using polyclonal or monoclonal antibodies specifically recognizing the said PLGF polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific PLGF polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific PLGF polypeptides or the aforementioned types of variants as long as these fragments have the essential immunological and biological properties as referred to above. Such fragments may be, e.g., degradation products or splice variants of the PLGF polypeptides. Further included are variants which differ due to posttranslational modifications such as phosphorylation or myristylation.

Determining the amount of the polypeptides (i.e. PLGF) referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a polypeptide can be achieved by all known means for determining the amount of a peptide in a sample. Said means comprise immunoassay devices and methods which may utilize labeled molecules in various sandwich, competition, or other assay formats. Said assays will develop a signal which is indicative for the presence or absence of the polypeptide. Moreover, the signal strength can, preferably, be correlated directly or indirectly (e.g. reverse- proportional) to the amount of polypeptide present in a sample. Further suitable methods comprise measuring a physical or chemical property specific for the polypeptide such as its precise molecular mass or NMR spectrum. Said methods comprise, preferably, biosensors, optical devices coupled to immunoassays, biochips, analytical devices such as mass-spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a polypeptide comprises the steps of (a) contacting a cell capable of eliciting a cellular response the intensity of which is indicative of the amount of the polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, another polypeptide, or a small molecule. The expression or substance shall generate an intensity signal which correlates to the amount of the polypeptide.

Also preferably, determining the amount of a polypeptide comprises the step of measuring a specific intensity signal obtainable from the polypeptide in the sample. As described above, such a signal may be the signal intensity observed at an mass to charge (m/z) variable specific for the polypeptide observed in mass spectra or a NMR spectrum specific for the peptide or polypeptide.

Determining the amount of a polypeptide may, preferably, comprises the steps of (a) contacting the polypeptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, another polypeptide, nucleic acid, or small molecule, binding to the polypeptide described herein. Preferred ligands include antibodies, nucleic acids, polypeptides such as receptors or binding partners for the polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Nonspecific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.
First, binding of a ligand may be measured directly, e.g. by NMR or surface plasmon resonance.
Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.
Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a polypeptide may be, also preferably, determined as follows: (a) contacting a solid support comprising a ligand for the polypeptide as specified above with a sample comprising the polypeptide and (b) measuring the amount polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide, the relative amount or concentration of the said polypeptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

It is to be understood that the physiological amounts for PLGF in a population of subjects will statistically vary. Thus, the said population will exhibit a range of PLGF amounts. The term "upper limit of normal" or "ULN" as used herein refers to an amount of PLGF which represents the average upper limit amount of PLGF to be found in an apparently healthy population of subjects. The subjects are, preferably, of the same species as the subject to be investigated and, even more preferably, of the same ethnical background. How to determine the ULN is well known in the art and has been carried out for various polypeptides already. Particularly, a suitable range for an ULN can be derived from the amounts found between the 25^{th} and 75^{th} percentiles. More preferably, the said ULN for PLGF is between 7 and 10 pg/ml, most preferably, 8 pg/ml. It will be understood that the ULN may vary due to statistics. Thus, variations in the ULN amount within standard deviations shall be also taken into account.

The term "calculating" as used herein refers to assessing the ratio of the amount of PLGF determined in the sample of the subject and the ULN. If the amount of PLGF determined in the sample is larger than the ULN, the ratio will be larger than one (1). The ratio will be less than 1, if the determined amount for PLGF is less than the ULN. The ratio will be 1 for an amount of PLGF determined in the sample being identical to the ULN. Moreover, it is to be understood that a ratio of 1 indicates a normal atherosclerotic load and, thus, normal risk for developing the severe complications associated with atherosclerosis. A ratio less than 1 indicates a reduced atherosclerotic load and, consequently, a reduced risk for developing the severe complications associated with atherosclerosis. A ratio larger than 1 indicates an increased atherosclerotic load and, as a result thereof, an increased risk for developing the severe complications associated with atherosclerosis.

Advantageously, it has been found in accordance with the present invention that PLGF is a suitable marker for assessing the atherosclerotic load in a subject and, thus, the risk for developing severe complications associated with atherosclerosis, preferably coronary heart diseases including angina pectoris, claudicato intermittens or steoke. Accordingly, instead of using expensive and time-consuming monitoring techniques such as angiography which are associated with potentially severe side effects, the method of the present invention allows for a fast, reliable, cost-effective and safe assessment of the atherosclerotic load. In principle, it has been found that PLGF can be advantageously used for diagnosing the arteriosclerotic load in a subject and for predicting whether a subject has an increased risk for developing angina pectoris, claudicato intermittens or stroke. It is to be understood that in addition to PLGF, further biomarkers can be determined in the methods of the present invention. Specifically, cardiac troponins, preferably troponin T or I, as well as natriuretic peptide, preferably NT-proBNP, can be determined in order to further evaluate the atherosclerotic load with respect to potential cardiovascular implications. The angiogenic status in the subject may also be taken into account by determining biomarkers which indicate a pro-angiogenic status, preferably, endoglin an soluble Flt-1.

The definitions and explanations of the terms given above apply mutatis mutandis for the preferred methods, the devices and kits referred to in the following.

The present invention further relates to a method for identifying a subject in need of prevention or therapy of arteriosclerosis, the method comprises the steps of the aforementioned method and the further step of identifying a subject in need of prevention or therapy of arteriosclerosis based on an increased arteriosclerotic load.

The term "prevention or therapy of arteriosclerosis" refers to drug-based therapies as well as nutritional diets or lifestyle recommendations. Preferred therapies are drugs against hypertension, lipid lowering drugs, preferably statins, aspirin, beta-blockers, ACE inhibitors, anticoagulation drugs, estrogen replacement, drugs against diabetes. Lifestyle recommendations include recommendations on smoking, body weight and exercise.

The present invention also relates to a device for diagnosing the arteriosclerotic load of a subject comprising:
a) means for determining PLGF in a sample of said subject; and
b) means for calculating the ratio of the determined amount from the means of a) and the upper limit of normal for PLGF, wherein
(i) a ratio of 1 indicates a normal arteriosclerotic load;
   (ii) a ratio less than 1 indicates a reduced arteriosclerotic load; and
   (iii) a ratio larger than 1 indicates an increased arteriosclerotic load.

The term "device" as used herein relates to a system of means comprising at least the aforementioned means operatively linked to each other as to allow the prediction. Preferred means for determining the amount of a PLGF polypeptide as well as means for carrying out the calculation are disclosed above in connection with the method of the invention. How to link the means in an operating manner will depend on the type of means included into the device. For example, where means for automatically determining the amount of the polypeptides are applied, the data obtained by said automatically operating means can be processed by, e.g., a computer program in order to obtain the desired results. Preferably, the means are comprised by a single device in such a case. Said device may accordingly include an analyzing unit for the measurement of the amount of the polypeptides in an applied sample and a computer unit for processing the resulting data for the evaluation. The computer unit, preferably, comprises a database including the stored ULN reference amounts or values thereof recited elsewhere in this specification as well as a computer-implemented algorithm for carrying out a ratio calculation of the determined amounts for the polypeptides and the the stored ULN reference amounts of the database. Computer-implemented as used herein refers to a computer-readable program code tangibly included into the computer unit. The person skilled in the art will realize how to link the means without further ado. Preferred devices are those which can be applied without the particular knowledge of a specialized clinician, e.g., electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the polypeptides, Plasmon surface resonace devices, NMR spectrometers, mass- spectrometers etc.) and/or evaluation units/devices referred to above in accordance with the method of the invention.

Finally, the present invention relates to a kit adapted for carrying out the aforementioned methods comprising
a) means for determining PLGF in a sample of said subject; and
b) means for calculating the ratio of the determined amount from the means of a) and the upper limit of normal for PLGF, wherein
   (i) a ratio of 1 indicates a normal arteriosclerotic load;
   (ii) a ratio less than 1 indicates a reduced arteriosclerotic load; and
   (iii) a ratio larger than 1 indicates an increased arteriosclerotic load.

The term "kit" as used herein refers to a collection of the aforementioned means, preferably, provided separately or within a single container. The components of the kit may be comprised by separate vials (i.e. as a kit of separate parts) or provided in a single vial. Moreover, it is to be understood that the kit of the present invention is to be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Example: Determination of PLGF in patients suffering from complications associated with atherosclerosis

296 patients suffering from a stable coronary artery disease, 51 patients suffering from a peripheral arterial occlusive disease as well as 149 clinically healthy patients were analyzed for the plasma levels for the angiogenesis biomarkers PLGF, soluble (s)Flt-1, endoglin as well as the organ specific biomarkers NT-proBNP and troponin T (sensitive test).

Plasma levels of PlGF, sFLT1, and Endoglin were determined using the commercially available Immunoassays "Quantikine" (Catalog numbers DVR100B, DPG00 and DNDG00) from R & D Systems, USA. NT-proBNP and sensitive Troponin T plasma levels were detected by the corresponding commercial Elecsys^{™} assays (Roche Diagnostics).

PLGF was found to correlate with the atherosclerotic load. The highest amounts for PLGF were found in patients suffering from the peripheral arterial occlusive disease which is indicative for a rather progressive atherosclerosis with a high atherosclerotic load. Stable coronary artery diseases usually result from less progressive atherosclerosis and are, thus indicative for a lower atherosclerotic load. These patients showed somewhat lower amounts for PLGF. The physiological amounts of PLGF are the lowest amounts and are found in the clinically healthy control subjects. The results are summarized in the following table:

| Biomar ker | PLGF pg/ml | | | sFlt1 pg/ml | | | Endoglin ng/ml | | | NT-proBNP pg/ml | | | Sens. Troponin T pg/ml | | |
|---|---|---|---|---|---|---|---|---|---|---|---|---|---|---|---|
| | H | CAD | PAD | H | CAD | PAD | H | CAD | PAD | H | CAD | PAD | H | CAD | PAD |
| 75th perc. | 10,13 | 15,88 | 19,79 | 216,54 | 124,02 | 113,11 | 5,45 | 5,03 | 4,68 | 67,58 | 928,8 | 508,28 | 0,1 | 23,07 | 16,13 |
| median | 7,8 | 11,35 | 18,38 | 141,99 | 94,8 | 95,29 | 4.78 | 4,38 | 3.96 | 37,25 | 266,05 | 213,9 | 0,1 | 6,64 | 8,64 |
| 25th perc. | 6,99 | 6,08 | 15,97 | 78,04 | 75,07 | 82,99 | 4,17 | 3,86 | 3,21 | 18,45 | 95,5 | 66,27 | 0,1 | 0,1 | 0,1 |

| | | | | | | | | | | | | | | | |
|---|---|---|---|---|---|---|---|---|---|---|---|---|---|---|---|
| H: clinically healthy subjects, n=149 CAD: coronary artery diseases subject, n=296 PAD: peripheral arterial occlusive disease, n=51 | | | | | | | | | | | | | | | |

## Claims

1. A method for diagnosing the arteriosclerotic load of a subject comprising:
c) determining the amount of PLGF in a sample of a subject; and
d) calculating the ratio of the determined amount and the upper limit of normal for PLGF, wherein
(i) a ratio of 1 indicates a normal arteriosclerotic load;
(ii) a ratio less than 1 indicates a reduced arteriosclerotic load; and
(iii) a ratio larger than 1 indicates an increased arteriosclerotic load.

2. The method of claim 1, wherein said upper limit of normal for PLGF is between 7 and 10 pg/ml.

3. The method of claim 1 or 2, wherein an increased arteriosclerotic load further indicates an increased risk of developing angina pectoris, claudicato intermittens or stroke.

4. The method of any of claims 1 to 3, wherein said subject is a human.

5. A method for identifying a subject in need of prevention or therapy of arteriosclerosis, the method comprises the steps of the method of any one of claims 1 to 4 and the further step of identifying a subject in need of prevention or therapy of arteriosclerosis based on an increased arteriosclerotic load.

6. Use of PLGF for diagnosing the arteriosclerotic load in a subject.

7. Use of PLGF for predicting whether a subject has an increased risk for developing angina pectoris, claudicato intermittens or stroke.

8. A device for diagnosing the arteriosclerotic load of a subject comprising:
a) means for determining PLGF in a sample of said subject; and
b) means for calculating the ratio of the determined amount from the means of a) and the upper limit of normal for PLGF, wherein
(i) a ratio of 1 indicates a normal arteriosclerotic load;
(ii) a ratio less than 1 indicates a reduced arteriosclerotic load; and
(iii) a ratio larger than 1 indicates an increased arteriosclerotic load.

9. A kit adapted for carrying out the method of any one of claims 1 to 5 comprising
a) means for determining PLGF in a sample of said subject; and
b) means for calculating the ratio of the determined amount from the means of a) and the upper limit of normal for PLGF, wherein
(i) a ratio of 1 indicates a normal arteriosclerotic load;
(ii) a ratio less than 1 indicates a reduced arteriosclerotic load; and
(iii) a ratio larger than 1 indicates an increased arteriosclerotic load.
